# EUROPEAN PATENT SPECIFICATION

(11) **EP 0 535 048 B1**
(45) Date of publication and mention of the grant of the patent: **02.04.1997**
(21) Application number: 91911030.4
(22) Date of filing: 30.05.1991
(51) Int. Cl.: C07K 14/00, C12N 9/20, C12N 9/16, C12N 15/55, A61K 38/46

(54) **USE OF DERIVATIVES OF HUMAN BILE-SALT STIMULATED LIPASE FOR THE PREPARATION OF MEDICAMENTS**
VERWENDUNG VON DERIVATEN DURCH MENSCHLICHES GALLENSALZ STIMULIERTER LIPASE ZUR HERSTELLUNG VON MEDIKAMENTEN
UTILISATION DES DERIVES DE LA LIPASE STIMULEE PAR LES SELS BILIAIRES DE L'HOMME POUR LA PREPARATION D'UN MEDICAMENT

(30) Priority: 01.06.1990 SE 9001985
(43) Date of publication of application: 07.04.1993
(73) Proprietor: Astra Aktiebolag, 151 85 Södertälje (SE)
(72) Inventor: BJURSELL, Gunnar, S-433 31 Partille (SE); BLÄCKBERG, Lars, S-902 44 Ume (SE); CARLSSON, Peter, S-414 59 Göteborg (SE); ENERBÄCK, Sven, S-431 69 Mölndal (SE); HERNELL, Olle, S-902 40 Ume (SE); NILSSON, Jeanette, S-413 14 Göteborg (SE); OLIVECRONA, Thomas, S-902 45 Ume (SE)
(74) Representative: Hjertman, Ivan T.
(86) International application number: SE9100381
(87) International publication number: WO9118923

(56) References cited:
- WO-A-85/00381
- DIALOG INFORMATION SERVICES, File 155, Medline 67-91; J. NILSSON et al., AN 91- 006144

## Description

### Field of the invention

The present invention relates to the use of an enzyme, known i.a. as human bile salt-stimulated lipase, for the manufacture of a medicament for the treatment of a pathological condition related to exocrine pancreatic insufficiency.

### Background to the invention

The human lactating mammary gland synthesizes and secretes with the milk a bile salt-stimulated lipase (BSSL) [1] that, after specific activation by primary bile salts [2, 57, 58], contributes to the breast-fed infant's endogenous capacity of intestinal fat digestion [3-5]. This enzyme, which accounts for approximately 1% of total milk protein [6], is a non specific lipase; in vitro it hydrolyses not only tri-, di- and monoacylglycerols, but also cholesteryl-, and retinyl esters, and lysophosphatidylglycerols [7-10]. Furthermore, its activity is not restricted to emulsified substrates, but micellar and soluble substrates are hydrolyzed at similar rates [11].
BSSL is not degraded during passage with the milk through the stomach, and in duodenal contents it is protected by bile salts from inactivation by pancreatic proteases such as trypsin and chymotrypsin [2,11]. It is, however, inactivated when the milk is pasteurized, e.g. heated to 62.5°C, 30 min. [12]. Model experiments in vitro suggest that the end products of triacylglycerol digestion are different in the presence of BSSL [5,7]. Due to lower intraluminal bile salt concentrations during the neonatal period [13,14] this may be beneficial to product absorption [5,15].
The carboxylic ester hydrolase (CEH) of human pancreatic juice [16] seems functionally to be identical, or at least very similar, to BSSL [8]. They also share common epitopes [8,17], have identical N-terminal amino acid sequences [17] and are inhibited by inhibitors of serine esterases, e.g. eserine and diisopropylfluorophopsphate [6,8,16]. It has been hypothesized that the two enzymes are products of the same gene [18,19]. The observed molecular size difference [8,19] could be explained by different patterns of glycosylation, as recently suggested [17].

Dietary lipids is an important source of energy. The energy-rich triacylglycerols constitute more than 95% of these lipids. Some of the lipids, e.g. certain fatty acids and the fat soluble vitamins, are essential dietary constituents. Before gastro-intestinal absorption the triacylglycerols as well as the minor components, i.e. esterified fat-soluble vitamins and cholesterol, and diacylphosphatidylglycerols, require hydrolysis of the ester bonds to give rise to less hydrophobic, absorbable products. These reactions are catalyzed by a specific group of enzymes called lipases. In the human adult the essential lipases involved are considered to be gastric lipase, pancreatic colipase-dependent lipase (tri- and diacylglycerol hydrolysis), pancreatic phospholipase A2 (diacylphosphatidylglycerols) and carboxylic ester hydrolase (cholesteryl- and fat soluble vitamin esters). In the breast-fed newborn bile salt-stimulated lipase plays an essential part in the hydrolysis of several of the above mentioned lipids. Together with bile salts the products of lipid digestion form mixed micelles from which absorption occurs (3-5).

Common causes of lipid malabsorption, and hence malnutrition, are reduced intraluminal levels of pancreatic colipase-dependent lipase an/or bile salts. Typical examples of such lipase deficiency are patients suffering from cystic fibrosis, a common genetic disorder resulting in a life-long deficiency in some 80% of the patients, and chronic pancreatitis, often due to chronic alcoholism.

The pancreatic and liver functions are not fully developed at birth, most notably in infants born before term. Fat malabsorption, for physiological reasons, is a common finding and thought to result from low intraluminal pancreatic colipase-dependent lipase and bile salt concentrations (3,4,13-15). However, because of BSSL, such malabsorption is much less frequent in breast-fed infants than in infants fed pasteurized human milk of infant formulas (3-5, 12, 59, 60, 61). This is one reason why it has been advocated that newborn infants, particularly preterm infants, that cannot be fed their own mothers milk should be fed non-pasteurized milk from other mothers (12).

The present treatment of patients suffering from a deficiency of pancreatic lipase is the oral administration of very large doses of a crude preparation of porcine pancreatic enzymes. Colipase-dependent pancreatic lipase is inactivated by low pH. Such conditions are prevalent in the stomach, with the result that orally administered pancreatic lipase is virtually completely inactivated on the passage through the stomach to the gut. Therefore, this effect cannot be completely overcome by the use of large doses of enzyme. The large doses administered are inadequate for most patients, and the preparations are impure and unpalatable. Certain tablets have been formulated which pass through the acid regions of the stomach and discharge the enzyme only in the relatively alkaline environment of the jejunum. However, many patients suffering from pancreatic disorders have an abnormally acid jejunum and such tablets may fail to discharge the enzyme and may therefore be ineffective. Moreover, since the preparations presently on the market are of a non-human source there is a risk of immunoreactions that may cause harmful effects to the patients or result in reduced therapy efficiency.
A further drawback with the present preparations is that their content of other lipolytic activities than colipase-dependent lipase are not stated. In fact, most of them contain very low levels of CEH/BSSL-activity. This may be one reason why many patients, suffering from cystic fibrosis in spite of supplementation therapy, suffer from deficiencies of fat soluble vitamins and essential fatty acids.
Thus, there is a great need for products with properties and structure derived from human lipases and with a broad substrate specificity, which products may be orally administered to patients suffering from deficiency of one or several of the pancreatic lipolytic enzymes. The products to be used according to the present invention fulfil this need by themselves or in combination with other lipases or in combination with preparations containing other lipases. Furthermore, for some human infants there is an obvious need to improve fat utilization from conventional infant formulas, or pasteurized human milk from so-called milk banks. BSSL has several unique properties that makes it ideally suited for substitution and supplementation therapy: It has been designed by nature for oral administration. Thus, it resists passage through the stomach and is activated in contents of the small intestine.
Its specific activation mechanism should prevent hazardous lipolysis of food or tissue lipids during storage and passage to its site of action.
Due to its broad substrate specificity it has the potential to, on its own, mediate complete digestion of most dietary lipids, including the fat soluble vitamin esters. BSSL may be superior to pancreatic colipase-dependent lipase to hydrolyze ester bonds containing long-chain polyunsaturated fatty acids.
In the presence of gastric lipase and in the absence of, or at low levels of colipase-dependent lipase BSSL can ascertain a complete triacylglycerol digestion in vitro even if the bile salt levels are low such as in newborn infants. In the presence of BSSL the end products of triacylglycerol digestion become free fatty acids and free glycerol rather than free fatty acids and monoacylglycerol generated by the other two lipases (5). This may favor product absorption particularly when the intraluminal bile salt levels are low (3,15).
From a historical point of view infant formulas have been developed, and improved, from the concept that their composition should be as similar to that of human milk as possible. It is desirable to supplement such formulas.

The utilization for supplementation, substitution or therapy of bile salt-stimulated lipases (BSSL), or of proteins with the essential functions of BSSL, requires however access to quantities of the product on a large technical scale. It is not possible in factory scale to rely on natural sources such as milk as starting material. Besides the problem mentioned above with inactivation of BSSL during pasteurization, there is the additional risk of contamination of material from a natural source with infectious agents, e.g. vira such as HIV virus and CMV.
There is, accordingly, a need for large scale access to products having BSSL properties.

Prior art references are given later in this specification.

### The invention

The present invention is based on the cloning of cDNA coding for BSSL derived from human mammary gland. We have also isolated, from human pancreas, a partial cDNA coding for CEH. Deduced amino acid sequences from the human cDNA's and comparison with CEH from other species, support the interpretation that BSSL and CEH are identical.

As will be further detailed below, it was surprisingly found that the structure of the protein as deduced from the cDNA sequence is quite different from the structure of other lipases. The structure proved unexpectedly to be more like the structure of typical esterases, such as cholinesterase.

With reference to Figure II and Figure VII, products to be used according to the invention are:
a) a protein as defined by the amino acid sequence 1-722 in Fig. VII,
b) a protein as defined by the amino acid sequence 1-535 in Fig. VII,
c) a protein as defined by the amino acid sequence 1-278 in Fig. VII,
d) a protein as defined by the amino acid sequence 1-341 in Fig. VII,
e) a protein as defined by the amino acid sequence 1-409 in Fig. VII,
f) a protein as defined by the amino acid sequence 1-474 in Fig. VII,
g) combinations of proteins defined under b) - f) e.g. as defined by the amino acid sequence in positions 1-278, 279-341, 279-409, 279-474, 342-409, 342-474 and 536-722.
h) combinations of proteins defined under b) - g) in combination with one or more of the repeats according to Figure V,
i) a protein as defined under a) - h) possessing an additional, N-terminal amino acid, namely methionine, and functionally equivalent variants and mutants of the proteins defined in a) - i) above;
   It should be noted that the proteins under a, b, c, d, e, f, h and i above will not be identical in all respects to naturally occurring BSSL, but they will exhibit one or more of the critical functions of naturally occurring BSSL. Critical functions are given below.
j) a DNA sequence coding for the proteins defined in a, b, c, d, e, f, h and i above.
k) a DNA sequence according to Fig. II, defined by the following nucleotide numbers in Fig. II:
   a) a DNA sequence 151-2316 according to Fig. II, coding for the protein defined by the amino acid sequence 1-722 in Fig. VII,
   b) a DNA sequence 151-1755 according to Fig. II, coding for the protein defined by the amino acid sequence 1-535 in Fig. VII,
   c) a DNA sequence 151-985 according to Fig. II, coding for the protein defined by the amino acid sequence 1-278 in Fig. VII,
   d) a DNA sequence 151-1172 according to Fig. II, coding for the protein defined by the amino acid sequence 1-341 in Fig. VII,
   e) a DNA sequence 151-1376 according to Fig. II, coding for the protein defined by the amino acid sequence 1-409 in Fig. VII,
   f) a DNA sequence 151-1574 according to Fig. II, coding for the protein defined by the amino acid sequence 1-474 in Fig. VII,
   g) a DNA sequence 986-1172 according to Fig. II, coding for the protein defined by the amino acid sequence 279-341 in Fig. VII,
   h) a DNA sequence 986-1376 according to Fig. II, coding for the protein defined by the amino acid sequence 279-409 in Fig. VII,
   i) a DNA sequence 986-1574 according to Fig. II, coding for the protein defined by the amino acid sequence 279-474 in Fig. VII,
   j) a DNA sequence 1173-1376 according to Fig. II, coding for the protein defined by the amino acid sequence 342-409 in Fig. VII,
   k) a DNA sequence 1173-1574 according to Fig. II, coding for the protein defined by the amino acid sequence 342-474 in Fig. VII.

Significant functions of proteins to be used according to the invention are
a) suitable for oral administration,
b) being activated by specific bile salts,
c) acting as a non-specific lipase in the contents of the small intestines, that is being able to hydrolyze lipids relatively independent of their chemical structure and physical state (emulsified, micellar, soluble).
d) Ability to hydrolyze triacylglycerols with fatty acids of different chain-length and different degree of unsaturation.
e) Ability to hydrolyze also diacylglycerol, monoacylglycerol, cholesteryl esters, lysophospatidylacylglycerol, and retinyl and other fat soluble vitamin-esters.
f) Ability to hydrolyze not only the sn-1(3) ester bonds in a triacylglycerol but also the sn-2 ester bond.
g) Ability to interact with not only primary but also secondary bile salts.
h) Dependency on bile salts for optimal activity.
i) Stability so that gastric contents will not affect the catalytical efficiency to any substantial degree.
j) Stability towards inactivation by pancreatic proteases, e.g. trypsin, provided bile salts are present.
k) Ability to bind to heparin and heparin derivatives, e.g. heparan sulphate.
l) Ability to bind to lipid-water interphases.
m) Stability to permit lyophilization.
n) Stability when mixed with food constituents such as in human milk, or milk formula.

The critical functions for supplementation, substitution, or therapy are these according to a), c), d), e), f), i), j) and 1). For other purposes, not all critical functions may be necessary.

For expression of the proteins indicated above, the appropriate DNA sequence indicated above will be inserted into a suitable vector which then is introduced into a suitable host organism. The said vector will also have to comprise appropriate signal and other sequences enabling the organism to express the desired protein.

### Suitable expression organisms:

With the recombinant DNA techniques it is possible to clone and express a protein of interest in a variety of prokaryotic and eukaryotic host organisms. Possible expression organisms are bacteria, simple eukaryots (yeast), animal cell cultures, insect cell cultures, plant cell cultures, plants and transgenic animals. Each individual system has its own particular advantages and disadvantages. The simple conclusion is that every gene to be expressed is a unique problem and no standard solution is available.

Commonly used bacterial systems are *E. coli, Bacillus subtilis, Streptomyces.* Commonly used yeasts are *Saccharomyces,* and *Pichia pastoris.* Commonly used animal cells are CHO cells and COS cells. Commonly used insect cell cultures are *Drosophila* derived cells.

Commonly used plant is the tobacco plant. Possible transgenic animals are goat and cow.

Possible bacterial vectors are pUC and protein A-vectors.
Possible yeast vector is pMA 91.
Possible insect vectors are derived from Baculo-virus.
Possible animal cell vectors are derived from SV/40. Possible plant vectors are derived from the Ti-plasmid.
In every system, both natural and synthetic promoters and terminators can be used.

It is understood that depending on the choice of expression system, the expressed protein may contain an additional N-terminal amino acid (methionine),contain a few extra amino acids, or be fused to a heterologous protein, (e.g. protein A), or differ from the naturally occurring protein with respect to glycosylation. Furthermore, the vectors may also contain signal sequences in order to export the protein to the periplasm or to the culture medium.
Thus, further aspects which are relevant for the expression of proteins to be used according to the invention are:
a) a vector comprising a DNA sequence coding for a protein as specified above,
b) a host organism comprising a DNA sequence as specified above,
c) a process for the production of a protein as specified above, by growing a host organism containing a vector as specified under a) above and isolating the protein.

Methods from purification are based on the expression system used (e.g. protein A/IgG) and/or on methods used for purification of the naturally occurring enzyme, as described in reference 6.

Aspects of the invention are:
- the use of a protein as specified above for the manufacture of a medicament for the treatment of a pathological condition related to exocrine pancreatic insufficiency,
- the use of a protein as specified above for the manufacture of a medicament for the treatment of cystic fibrosis,
- the use of a protein as specified above for the manufacture of a medicament for the treatment of chronic pancreatitis,
- the use of a protein as specified above for the manufacture of a medicament for the treatment of fat malabsorption of any etiology,
- the use of a protein as specified above for the manufacture of a medicament for the treatment of malabsoption of fat soluble vitamins,
- the use of a protein as specified above for the manufacture of a medicament for the treatment of fat malabsorption due to physiological reasons, e.g. in newborn infants.

The DNA sequence in Fig. II from position 151 up to and including position 2316 is the sequence coding for the entire protein. The sequence from position 2317 up to and including position 2415 is not translated to protein, but is included in exon d identified in Table 2 below.

### Experimental part

Abbreviations
aa, amino acid; bp, base pair; BSSL, bile salt-stimulated lipase; c-AMP, cyclic adenosine monophosphate; CEH, carboxylic ester hydrolase; Da, dalton; c ⁷GTP, 7-deaza-2-deoxyguanosine 5'triphosphate; EDTA, ethylene diamine tetraacetate; kb, kilobases; MOPS, 3-N-morpholinopropanesulfonic acid; nt, nucleotide; PAGE, polyacrylamide gel electrophoresis; SDS, sodium dodecyl sulfate; SSC, NaCl citrate, xGal, 5-bromo-4-chloro-3-indolyl-β-D-galactopyranoside.

Enzymes
Bile salt-stimulated lipase EC 3.1.1.3
Carboxylic ester hydrolase EC 3.1.1.1

### Material and Methods

### A. Enzyme and antibody preparation

BSSL was purified from human milk as previously described [6]. When used for antibody production the enzyme was further purified by SDS-PAGE. The protein band corresponding to the lipase was, after staining with Coomassie Brilliant blue, electroeluted from tne gel. Twentyfive µg of purified enzyme, together with an equal volume of Freund's complete adjuvant, was used fcr a first i.c. injection and the same amount of enzyme with incomplete adjuvant for the subsequent monthly booster injections. The rabbits were bled about two weeks after each booster and sera prepared and stored at -20 °C.

### B. Preparation of tryptic fragments and amino acid sequence analysis

Three mg of purified BSSL was dissolved in 1 ml of 0.1M Tris-Cl buffer, pH 8.5, containing 6M guanidinium hydrochloride and 2 mM EDTA. Dithioerythritol was added to 5mM. After incubation at 37 °C for 2h, 300 µl 50 mM iodoacetate was added. After 90 min incubation at 25 °C in darkness the reduced and carboxymethylated enzyme was desalted on a Sephadex G-25 column, equilibrated with 0.5M ammonium bicarbonate. Thirty µg of tosyl-L-phenylalanine chloromethane treated bovine trypsin (Worthington diagnostics system Inc., Freehold, NJ, USA) was added before lyophilization. The lyophilized protein was dissolved in 4 ml 0.1M ammonium bicarbonate and an additional 90 µg of trypsin was added. After 5h incubation at 37 °C the protein was again lyophilized. The tryptic digest was dissolved in 0.1% trifluoroacetic acid (2mg/ml). Three hundred µg of trypsinated BSSL was chromatographed on HPLC using a C-18 reversed phase column and eluted with a gradient of 0-50% acetonitrile in 0.1% trifluoroacetic acid. Peptide collection was monitored by continuous recording of the absorbance at 215 nm. Peptides to be sequenced were further purified by rechromatography using the same column with adjusted gradients. Samples of peptide fragments to be sequenced were dried under nitrogen to remove acetonitrile and applied to the sequencer. For N-terminal sequence analysis, native BSSL was dissolved in 0.1% acetic acid. Sequence analyzes were performed on an Applied Biosystems Inc. 477A pulsed liquid-phase sequencer and on-line PTH 120A analyzer with regular cycle programs and chemicals from the manufacturer. Initial and repetitive yields, calculated from a sequenced standard protein, β-lactoglobulin, were 47 and 97%, respectively.

### C. Isolation of RNA

Samples of human pancreatic adipose and lactating mammary gland tissues were obtained at surgery and immediately put into guanidinium thiocyanate (1-5 g in 50 ml). Total RNA was extracted as described by Chirgwin [20]. Poly(A)-RNA was prepared by chromatography on oligo-deoxythymidilate-(oligo(DT))-cellulose column [21].

### D. Construction and screening of cDNA libraries

Approximately 15 µg poly-adenylated RNA from human pancreas was denatured with methyl mercuric hydroxide [22] and primed with oligo (dT) ₁₂₋₁₈ primers (Pharmacia, Uppsala, Sweden), and reversely transcribed using standard procedures [23]. Second-strand synthesis was carried out according to Gubler and Hoffman [24], except that DNA ligase and 8-NAD were omitted, and the reaction temperature was set at 15 °C. Excess RNA was digested with RNAse A (50µg/ml), and the double-stranded cDNA was treated with EcoRI methylase [25]. Ends were blunted with Klenow enzyme. After ligation to EcoRI linkers and cleavage with EcoRI the cDNA was fractionated on a Sepharose 4B-Cl column. The void volume fraction was precipitated with ethanol and the cDNA ligated into the EcoRI site of a phosphatase treated λgt11 vector [26]. In vitro packing yielded more than 7x10⁵ recombinants.

A cDNA library from human mammary gland, derived from tissue obtained from a women at the eighth month of pregnancy, was purchased (Clontech Laboratories, Inc., Palo Alto, CA; USA).

Phages from the cDNA libraries were plated at 5x10⁴ plaque forming units per 120-mm dish. The antiserum was diluted to a ratio of 1:3200 and screening was performed according to Young and Davis [27]. Alkaline-phosphatase-conjugated goat-anti-rabbit antibodies were used as second antibodies (Bio-Rad, Richmond, CA USA). To isolate clones corresponding to the 5'-end of the mRNA, nucleic acid hybridization was done under standard conditions [23] using a subcloned fragment from one of the immunopositive clones as a probe.

### E. RNA analysis

Electrophoresis was carried out in a 1% agarose gel in 40mM MOPS buffer pH 7.0 after denaturation with glyoxal and dimethylsulfoxide [28]. Glyoxalated total RNA was then transferred to nitrocellulose filters [29]. The blots were probed with subclones of BSSL and CEH recombinants that were labelled by the oligo-labeling technique [30]. Prehybridization and hybridization were carried out with 50% formamid at 46 °C [23]. Posthybridization washes were performed at high stringency (0.1% SDS and 0.1xSSC at 60° C). (1xSSC, 0,15M NaCl, 0.0015M Na₃ citrate, pH 7.6).

### F. Nucleotide sequence

cDNA inserts from BSSL and CEH recombinants were either directly cloned into M3mp18 and mp19 after sonication and size fractionation or some of them were further subcloned into pTZ19R after digestions with PstI, BstXI, NarI, SmaI and AhaII. The nucleotide sequence was determined by the dideoxy chain-termination method [31]. The GC-rich repeats (see below) were also sequenced with TaqI polymerase and dc ⁷GTP. Both strands were sequenced. Sequence information was retrived from autoradiograms by use of the software MS-EdSeq as described by Sjöberg et al [55].

### G. Amino acid sequence predictions and homologies.

To predict the corresponding amino acid sequence of the cDNA inserts, codon usage of different reading frames was compared according to Staden and gave one open reading frame [32]. Homologies were searched for with the programs of the UWGCG software package [33].

### Results and Discussion

### A. Sequences of tryptic fragments and the N-terminus of BSSL

Trypsin digestion of purified BSSL resulted in approximately 50 fragments as judged by the number of peaks obtained during the HPLC-chromatography (Fig. I). The peaks were collected and the indicated peaks which could be isolated in a highly purified state and in reasonable quantities, were sequenced. The resulting sequences are shown in Table I. In addition the 30 most N-terminal residues were sequenced (Fig. II), and they confirm the previously reported sequence of Abouakil et al. (30 residues) [17].

### B. Nucleotide sequence of BSSL

For construction of the λgt11 cDNA library we used polyadenylated RNA from human pancreas. Initially four immunopositive clones were isolated, and then this pancreatic expression cDNA library was screened with antiserum against BSSL. Nucleotide sequence analysis of the four clones showed that they are in perfect agreement and correspond to the 3'-end of the mRNA. They all begin with a poly A tail and differ only in length; the longest insert, designated ACEH, spans 996 bp.
A cDNA library from human mammary gland was screened with antiserum, and the pancreas clone ACEH as probe. Positive clones were isolated from both screenings, which all originate from the 3'-end. The longest mammary gland clone, designated ABSSL, reaches 2100 bp upstream. It contains four of the sequenced tryptic fragments (Fig. II), but do not include the N-terminal amino acid sequence. To extend the sequence beyond the translation start, the mammary gland cDNA library was rescreened with a 118 bases long probe derived from the most 5'proximal part of ABSSL. One clone was isolated that continued a further 328 nucleotides upstream. It matched the N-terminal amino acid sequence, and contained the remaining tryptic fragment. As shown in Fig. II, the cDNA is 2428 nucleotides long and contains 81 bases upstream from the first ATG codon. The polyadenylation signal, AATAAA is located 13 nucleotides upstream from the poly A tail and the termination codon TAG was found at nucleotide 2317 followed by a 3'-untranslated region of 112 bp. A GC rich region consisting of 16 repeats of 33 bases was found in the 3'-end of the sequence between base 1756 and 2283. The nucleotide sequence of the repetition, shown in Fig. III, consists of six identical repetitions surrounded by ten repetitions with different number of substitutions that have probably occurred after several duplications. The low number of substitutions suggests that these repetitions have appeared late during evolution.

### C. Tissue distribution of expression

RNA from human lactating mammary gland, pancreas, adipose tissue and from a human hepatoma celline (HepG2) was analyzed by Northern blotting. The size of the messenger was determined to be approximately 2.5 kb in both lactating mammary gland and pancreas. No signal could be detected in the lanes with RNA extracted from HepG2 or adipose tissue (Fig. IV).
Since the mRNA used for the mammary gland library was obtained from a female in her 8th month of pregnancy, it is evident that transcription and probably translation of the BSSL gene is turned on before partus, in agreement with previous findings on BSSL secretion before partus [35]. See Figure IV.

### D. Amino acid sequence of BSSL

Assessed by SDS-PAGE the molecular mass has been reported to be 107-125 kDa [8,36] and by analytical ultracentrifugation to be 105 kDa [37]. The enzyme, as deduced from the cDNA, consists of 722 amino acid residues (Fig. II) which, giving a molecular mass of 76.271 Da, indicates that the enzyme contains at least 15-20% carbohydrate. The leader sequence is 23 residues long. A tentative active site serine residue is localized to serine-217 (Fig. V). The sequence around this serine accord with the consensus active site sequence of serine-hydrolases [38]. It has recently been proposed that basic residues found close to the active site serine may be involved in the cleavage of esterbonds in acylglycerols by lipases [39]. It is interesting to note that such residues are not present in BSSL. The single tentative N-glycosylation site is localized only seven residues from the serine. The degree of glycosylation [6,16] suggests that the enzyme contains 0-linked carbolydrate. There are numerous sites where such glycosylation could have occurred. The amino acid composition based on purified enzyme has shown a high content of proline residues [6]. The amino acid sequence obtained from cDNA confirms this. Moreover, most of the proline residues are localized in the 16 repeats of 11 residues each, constituting the main part of the C-terminal half of the enzyme.

### E. Comparison of the enzymes in mammary gland (BSSL) and pancreas (CEH)

BSSL of human milk and human pancreas CEH have previously been shown to be similar, if not identical. The present data strongly suggests that the two enzymes are products of the same gene. The nucleotide sequence of the cDNA clones shows that the pancreatic clone ACEH is identical with the mammary gland clone ABSSL from the poly A tail and 996 bases towards the 5'-end, including the sequence coding for the proline rich repeats. Northen blot gave a single band of 2.5 kb in RNA from pancreas and lactating mammary gland (Fig. IV). Genomic Southern blots further support the idea that only one gene codes for BSSL and CEH. The difference in mobility on SDS-PAGE between BSSL and CEH can be explained as a consequence of different glycosylation or differential splicing.

The similarity of BSSL to the rat and bovine enzymes (see below) and to results from genomics blots support the possibility that differential splicing cannot account for the mobility difference. Since the C-terminal sequence has not been confirmed on the protein level there is a less likely possibility that CEH may be processed by a proteolytic cleavage in the C-terminal end.

So far as we know pancreatic enzymes that obviously correspond to CEH have often been named after species and the particular substrates used to determine their respective activities; lysophospholipase, cholesteryl esterase, sterol ester hydrolase, non-specific lipase, carboxyl ester lipase and cholesteryl ester hydrolase. Available data are compatible with the view that all these activities described originates in one and the same functional entity [42,43]. This illustrates the broad substrate specificity and the relevance of designating them as non-specific lipases. When the sequence of human BSSL/CEH is compared to the sequence of lysophospholipase from fat pancreas [40] and cholesterol esterase from bovine pancreas [41] extensive similarities are found that extend about 530 residues from the N-terminal (Fig V); but they differ in the part of the molecule where the repeats occur. The rat enzyme has only four repeats and the bovine three. Hence the human enzyme is a considerable longer peptide.

Moreover, striking similarities were found between BSSL and a number of typical esterases, e.g. acetyl choline esterases from several species, including man and Drosophila, and carboxyl esterases (Fig. VI). These similarities were restricted to the N-terminal 300 residues of BSSL which includes the tentative active site serine-residue. A similarity to acetyl choline esterase has been predicted from the fact that BSSL is inhibited by typical choline esterase inhibitors [6, 8, 16]. With the possible exception of the rat liver carboxyl esterase [45), none of these similar enzymes has been show to have the same bile-salt dependency as BSSL; this suggests that the structural basis for this property resides in the C-terminal part of the protein. Moreover, BSSL can efficiently attack emulsified substrates which is not a known characteristic of the similar esterases. For this activity bile salt is a prerequisite.

The predicted sequence for human BSSL was compared with other well characterized mammalian lipases. Apart from the consensus sequence around the active site serine (G-X-S-X-G), no obvious similarities were found [44].

In addition to the similarities with other enzymes, there also significant similarities to one c-AMP dependent protein from Dictyostelium discoideum [46] as well as to thyroglobulin from several species (Fig VI) [47-49]. The similarities between BSSL and thyroglobulin, which comprise the active site region but not the active site itself, indicate that these highly concerved stretches of amino acids are of more generalized importance than merely supporting the enzymatic activity of esterases.

In conclusion, human milk BSSL consists of 722 amino acid residues. Available data strongly indicate that its peptide chain is identical to that of pancreatic CEH, and they are coded for by the same gene. The strongest evidence is that the nucleotide sequences of their 3 - ends and their N-terminal amino acid sequences are identical. The striking homologies found to rat pancreatic lysophospholipase and bovine pancreatic cholesterol esterase support the hypothesis that also these enzymes are functionally identical. However, as it has been suggested, the different molecular sizes found among species are not due to differences merely in glycosylation; instead they reflect a variable number of an eleven amino acid repeat. The similarity of the active site sequence between these esterases suggests that these proteins derive from a common ancestral gene.

With reference to Figures I-VII, the following legends are given.

### Figure I: Separation of the tryptic digest of BSSL on HPLC

Purified BSSL was treated with trypsin and chromatographed on HPLC as described in Materials and Methods. The indicated peaks were collected and purified further by a rechromatograph and their amino acid sequence determined.

### Figure II: The cDNA nucleotide sequence and the deduced amino acid sequence for human bile salt-stimulated lipase:

The cDNA is 2428 bases long. The N-terminal 23-codon sequence (nt82-150) starting with an ATG, is interpreted as a leader peptide since the N-terminal amino acid sequence of the mature protein starts at codon 24 (nt 151, Ala). The leader peptide is underlined. The sign * indicates the starting point of an exon. The sign * indicates the starting point of the repetition part.

### Figure III: The nucleotide sequence of the C-terminal GC-rich repetitions in the bile salt-stimulated lipase:

### Substitutions are indicated by a *.

### Figure IV: Northern blot hybridization

Northern blot analysis of total RNA isolated from human lactating mammary gland, pancreas, adipose tissue and a human hepatoma cell line (HepG2). Total RNA (10µg) from lactating mammary gland (lane A), pancreas (lane B), adipose tissue (lane C) and HepG2 (lane D) were electrophoresed in a 1% agarose gel in 40mM MOPS buffer at Ph 7.0 after denaturation of RNA in 1M glyoxal, 50% dimethylsulfoxide and 40mM MOPS. The glyoxalated RNA was then transferred to nitrocellulose paper for hybridization with [³² P] labeled BGSL cDNA (ABSSL).

### Figure V: Comparison of the deduced amino acid sequence from human milk BSSL, rat pancreatic lysophospholipase (Rat1p1) [40] and bovine pancreatic cholesterol esterase (Bovceh) [41]:

The serine residues involved in the active site are indicated by a *, and the # indicates the single possible N-glycosylation signal of the protein. The direct repeats of amino acid sequences are boxed. Matching sequences are denoted in capital letters, matching sequences between two enzymes are denoted in small letters and mismatching with a dot.

### Figure VI: Comparison of the primary structure of BSSL to other esterases, thyroglobuline and to one c-AMP dependent enzyme from Dictyostelium discoideum:

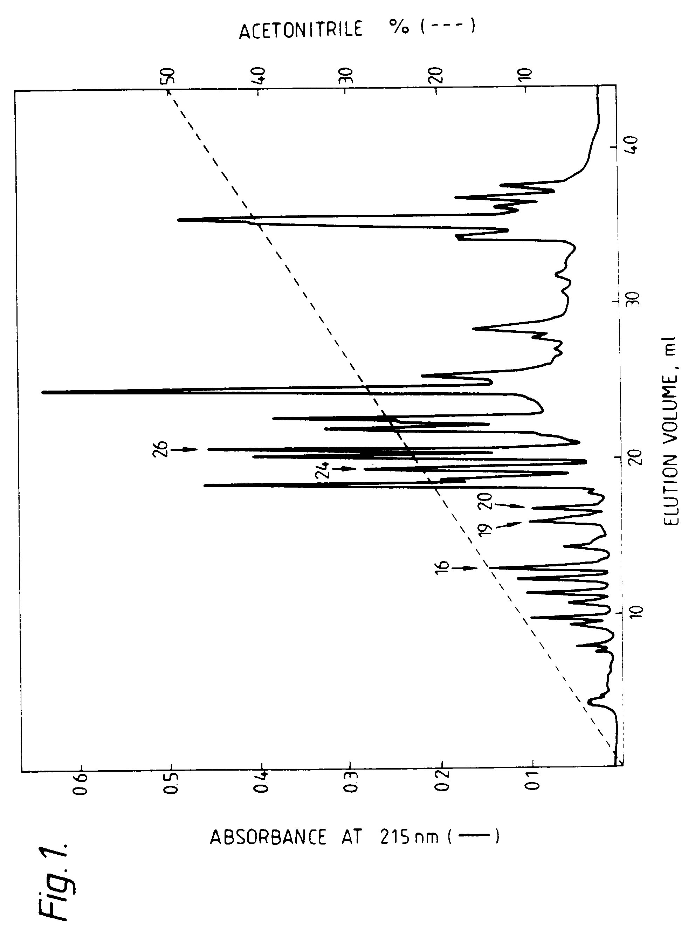

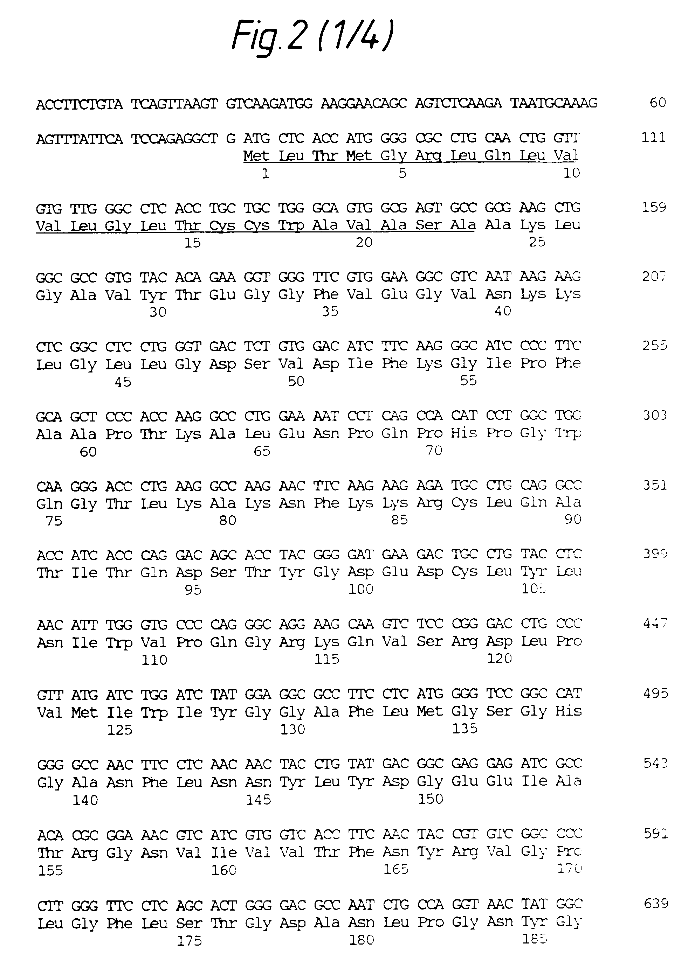

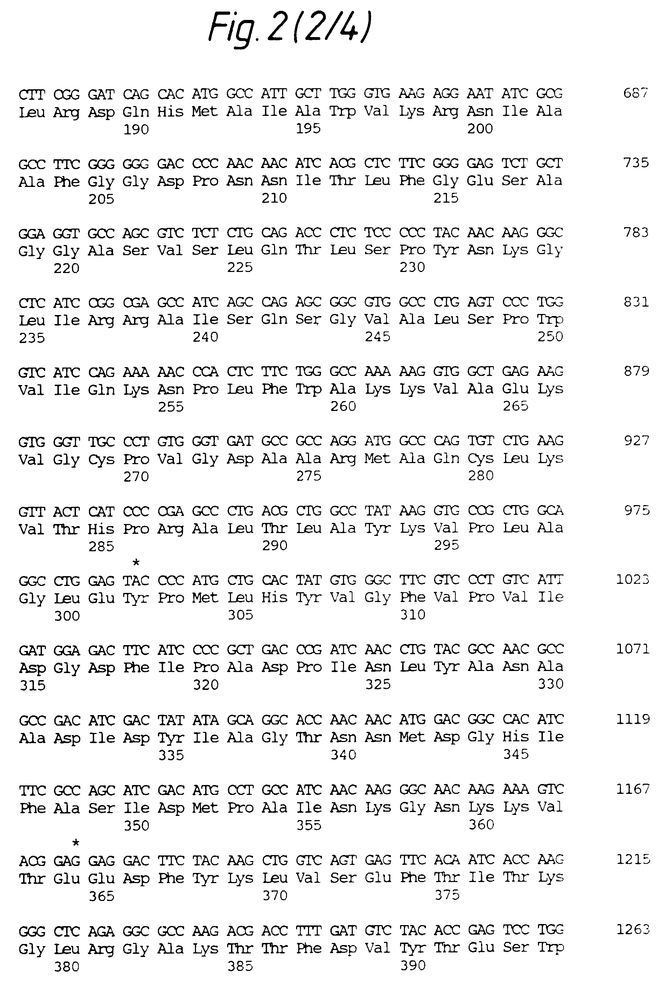

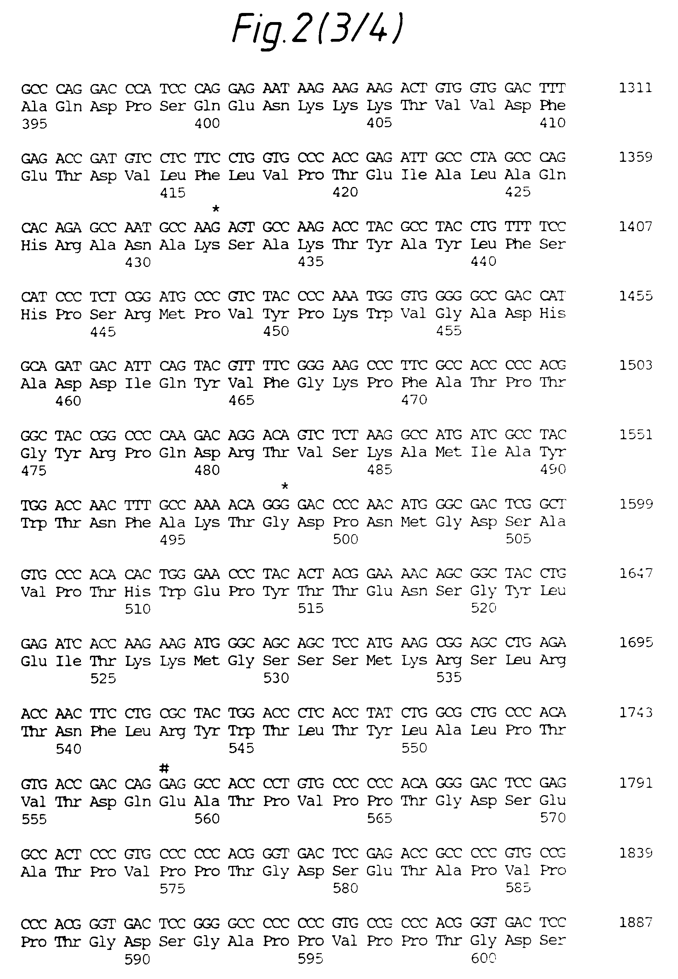

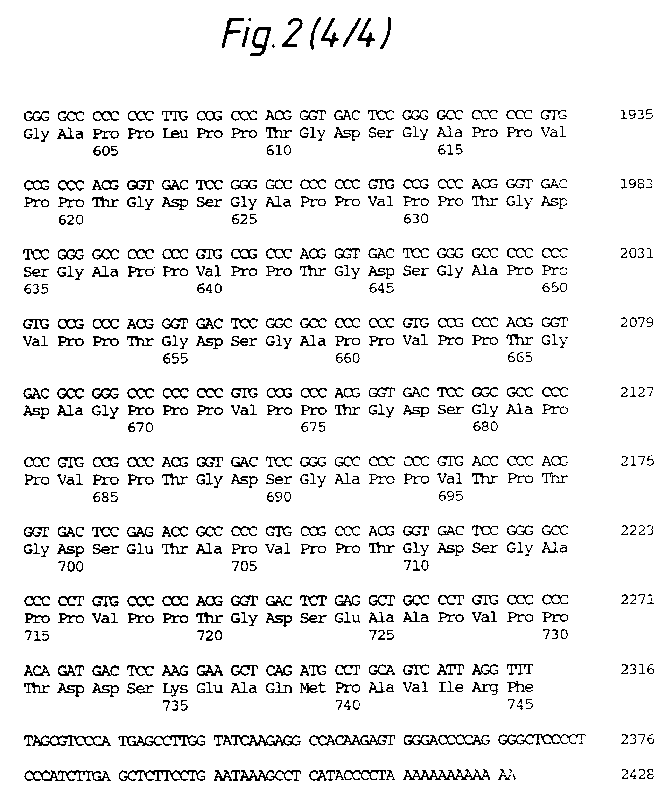

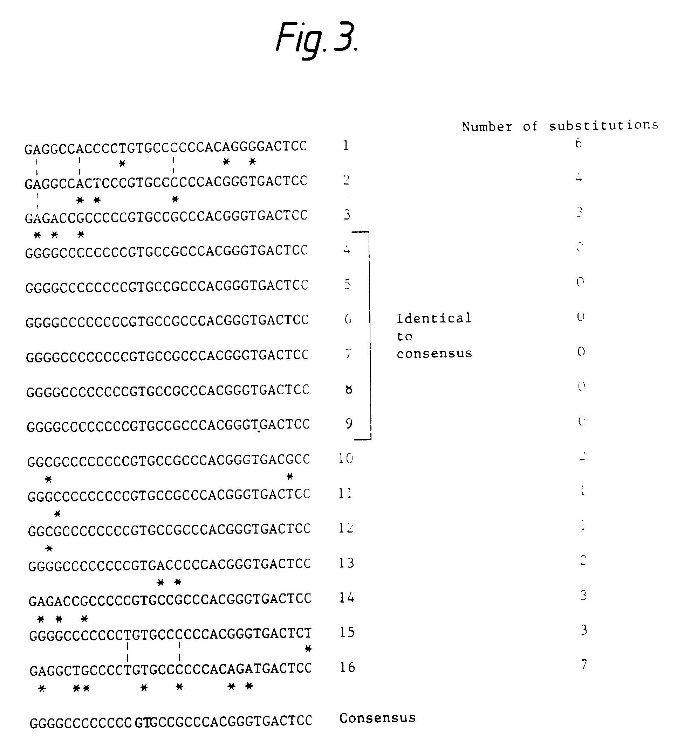

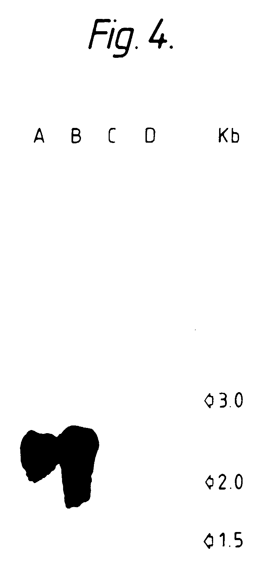

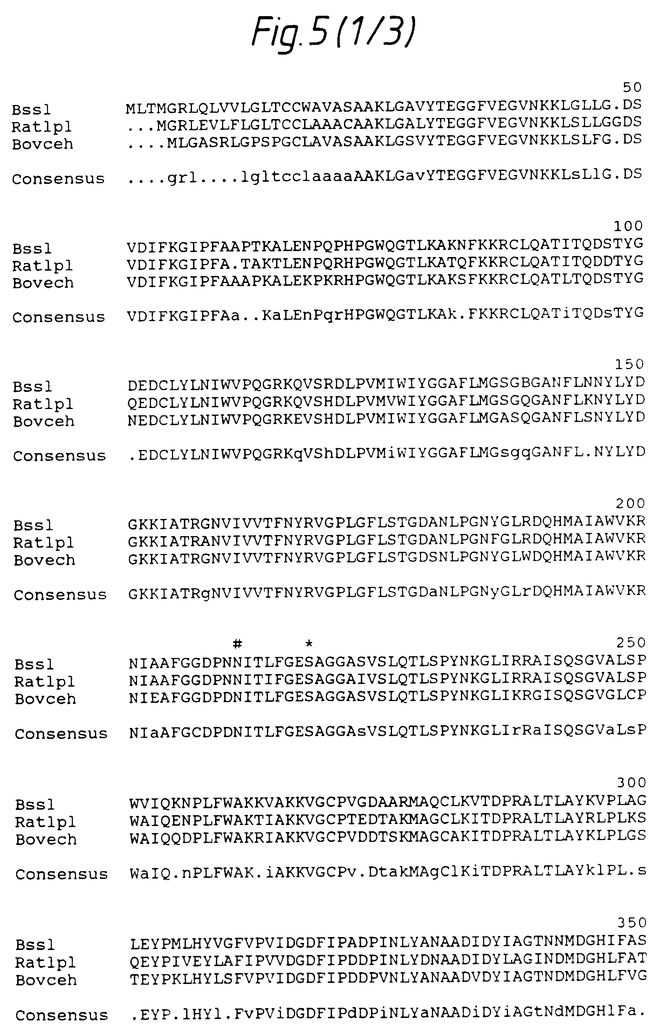

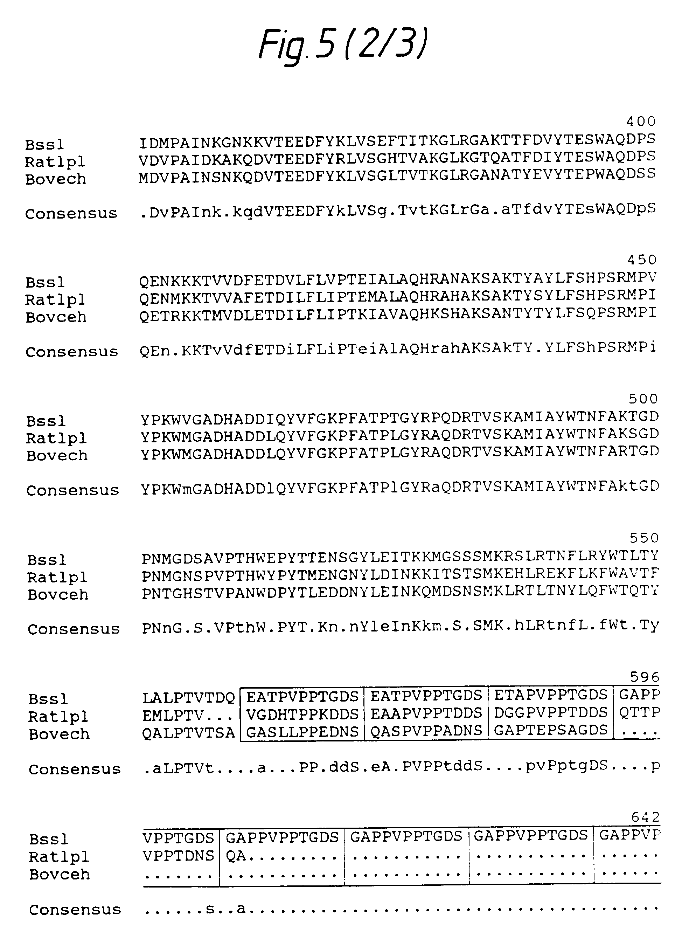

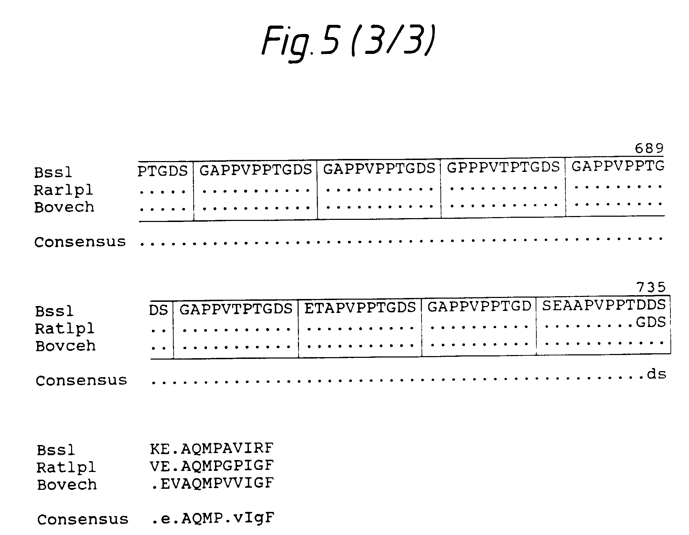

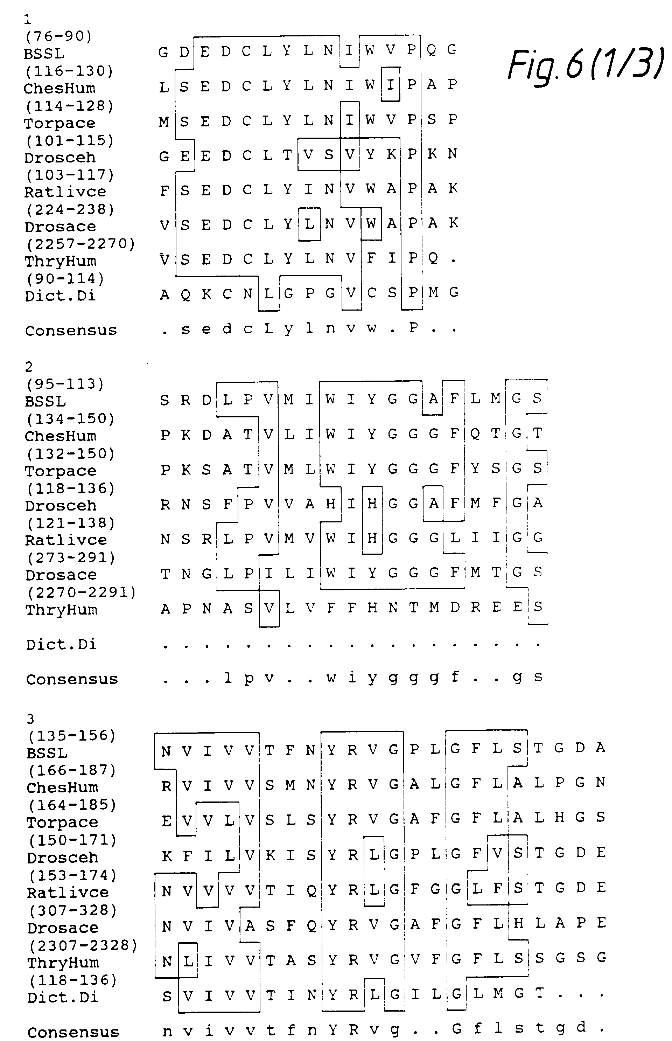

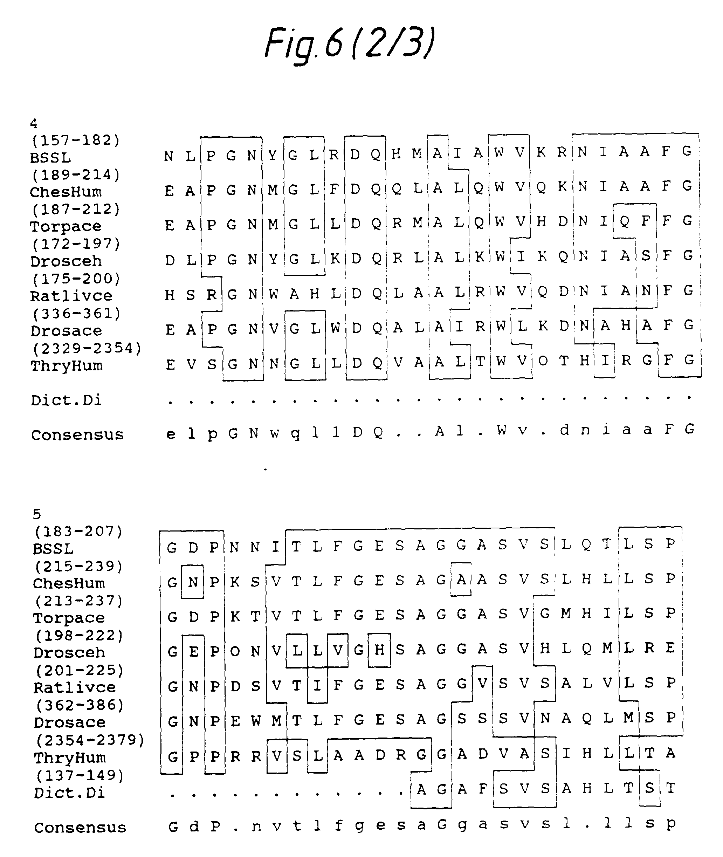

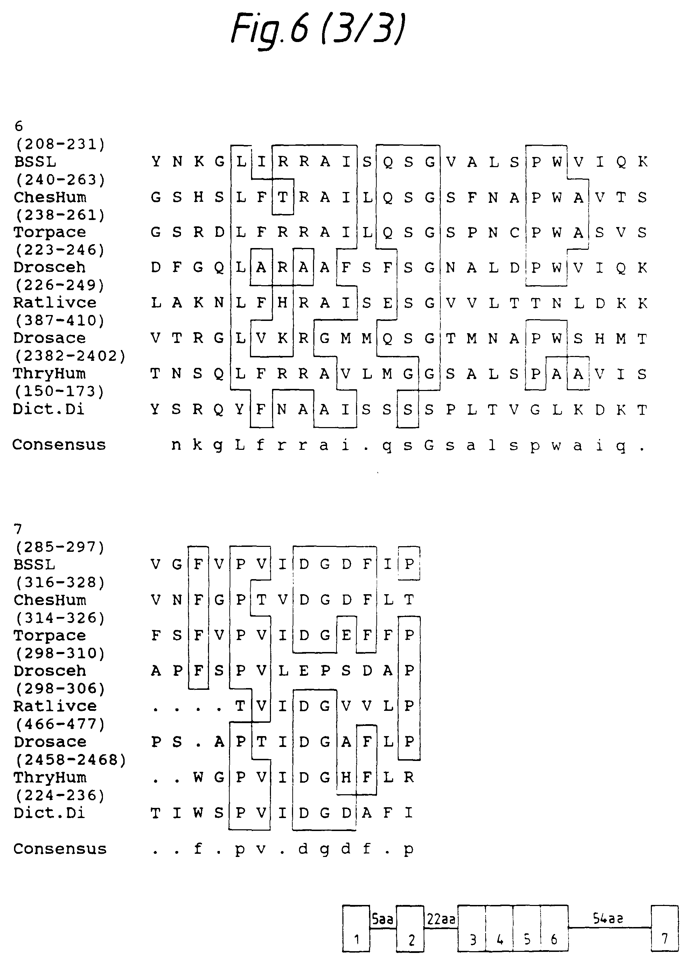

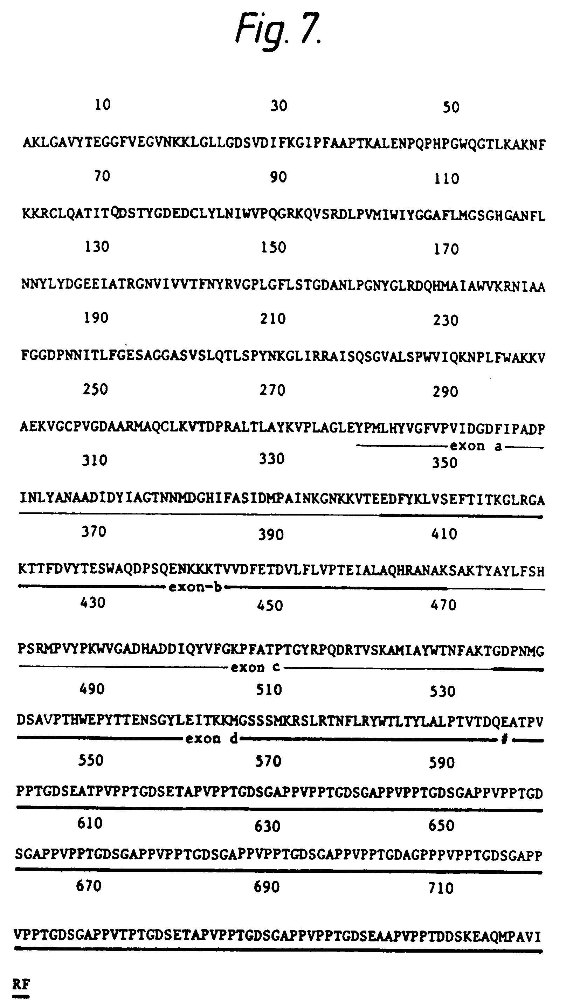

BSSL: bile salt stimulated lipase from human, Cheshum: cholinesterase from fetal human tissue [50], Torpace: acetylcholinesterase from Torpedo marmorata [51], Drosceh: carboxylic ester hydrolase from Drosophila melaogaster [52], Ratlivce: carboxyl esterase from rat liver [53], Drosace: acetylcholinesterase from Drosophila melaogaster [54], Thyrhum: thyroglobulin from human [49] and Dict.Di: c-AMP dependent enzyme from Dictyostelium discoideum [46]. There are 7 different domains that show similarities between the enzymes. Boxes enclose residues which are identical and small letters in the consensus sequence indicate identical residues in all the enzymes except for one. Dots indicate mismatches. The serine residue involved in the active site is indicated with *. The figure in the right hand corner shows how the domains are oriented.

### Figure VII:

gives the amino acid sequence 1 - 722 for the entire protein (one letter code) and indicates exons a, b, c, and d. The sign # indicates the starting point of the repetition part.

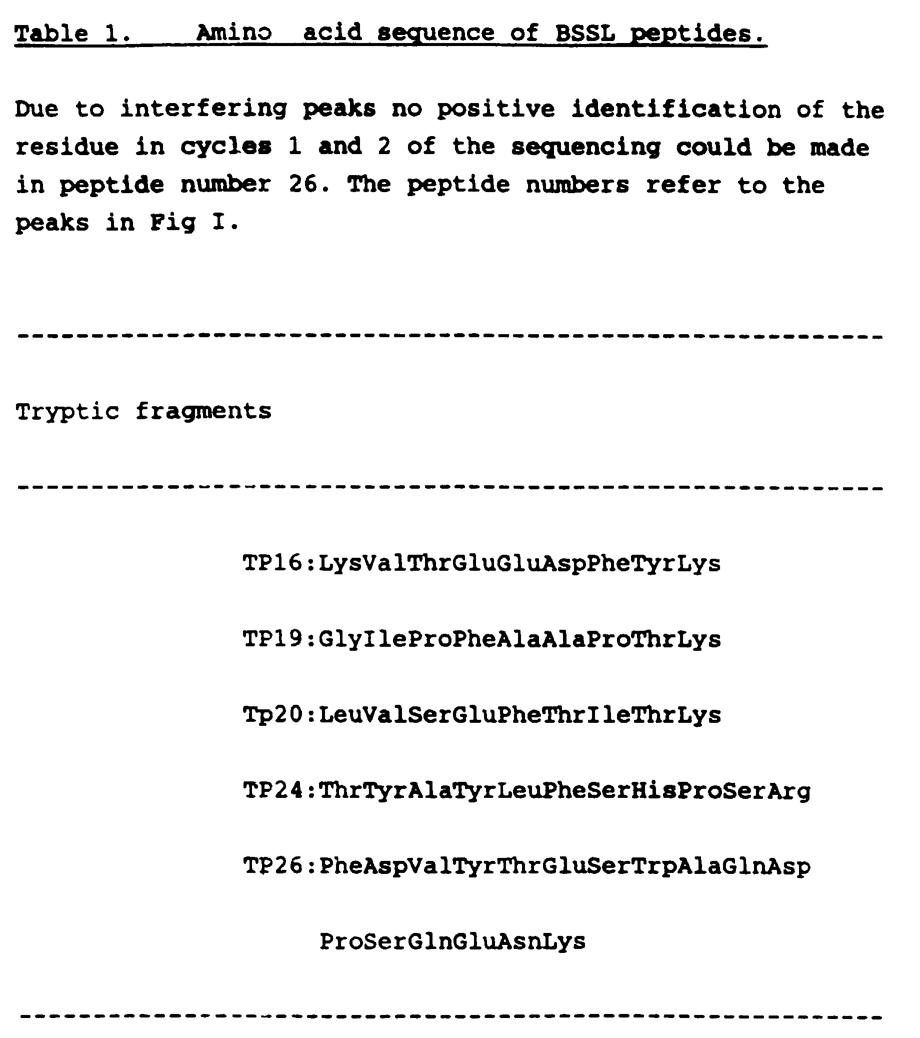

**Table 2:**

| **Identification of the exons a, b, c and d numbered as in Figures II and VII.** | | |
|---|---|---|
| exon | Location | |
| | between nucleotide number | between amino acid number |
| a | 986-1172 | 279-341 |
| b | 1173-1376 | 342-409 |
| c | 1377-1574 | 410-474 |
| d | 1575-2415 | 475-722 |
| | | |
| the entire protein | 151-2316 | 1-722 |
| the entire protein excluding repetitions | 151-1755 | 1-535 |

### References

1. Bläckberg, L., Ängquist, K.A, & Hernell, O. (1987) FEBS Lett. 217, 37-41.
2. Hernell, O. (1975) Eur. J. Clin. Invest. 5, 267-272.
3. Hernell, O., Bläckberg, L. & Bernbäck, S. (1988) In Perinatal nutrition (Lindblad, B.S., ed.) Bristol-Myers Nutrition symposia vol. 6, pp. 259272, Academic Press, New York.
4. Hernell, O, L. Bläckberg, B. Fredrikzon, and T. Olivecrona. 1981. Bile salt-stimulated lipase in human milk and lipid digestion in the neonatal period. In Textbook of gastroenterology and nutrition in infancy. E. Lebenthal, editor. Raven Press, New York. 465-471.
5. Bernbäck, S., Bläckberg, L. & Hernell, O. (1990) J. Clin. Invest. J. Clin. Invest. 221-226 (1990)
6. Bläckberg, L. & Hernell, O. (1981) Eur. J. Biochem 116, 221-225.
7. Hernell, O. & Bläckberg, L. (1982) Pediatr. Res. 16, 882-885.
8. Bläckberg, L. Lombardo, D., Hernell, O., Guy, O. & Olivecrona, T. (1981) FEBS Lett. 136, 284-288.
9. Fredrikzon, B., Hernell, O., Bläckberg, L. & Olivecrona, T. (1978) Pediatr. Res. 12, 1048-1052.
10. Wang, C.-S., Hartsuck, J.A. & Downs, D. (1988) Biochemistry 27, 4834-4840.
11. Bläckberg, L. & Hernell, O. (1983) FEBS Lett. 157, 337-341.
12. Björksten,B., Burman, L.G., deChateau, P., Fredrikzon, B., Gothefors, L. & Hernell, O. (1980) Br. Med. J. 201, 267-272.
13. Brueton, M.J., Berger, H.M., Brown, G.A., Ablitt, L., Iyangkaran, N. & Wharton B.A. (1978) Gut 19, 95-98.
14. Murphy, G.M. & Signer, E. (1975) Gut 15, 151-163.
15. Hernell, O., Staggers, J.E. & Carey, M.C. Biochemistry. (1990), 29:2041-2056.
16. Lombardo, D., Guy, O. & Figarella, C. (1978) Biochim. Biophys. Acta 527, 142-149.
17. Abouakil, N., Rogalska, E., Bonicel, J. & Lombardo, D. (1988) Biochim. Biophys. Acta 961, 299-308.
18. Hernell, O., Bläckberg, L. & Lindberg, T. (1988) in Textbook of Gastroenterology and Nutrition in infancy (Lebenthal, E., ed) pp. 209-217, Raven Press, New York.
19. Wang, C.-S. (1988) Biochem. Biophys. Res. Com. 155, 950-955.
20. Chirgwin, J.M. Przybyla, A.E., MacDonald R.J. & Rutter, W.J. (1979) Biochemistry 18, 5294-5299.
21. Aviv, H. & Leder, P. (1979) Proc. Natl. Acad. Sci. USA 69, 5201-5205.
22. Bailey, J.M. & Davidson, N. (1976) Anal. Biochem. 70, 75-85.
23. Maniatis, T., Fritsch, E.F. & Sambrook, J. (1982): Molecular Cloning. A Laboratory Manual. Cold Spring Harbor Laboratory, Cold Spring Harbor, New York.
24. Gubler, U. & Hoffman, B. J. (1983) Gene 25, 263-269.
25. Maniatis T., Haldison, R.C., Lacy, E., Lauer, J., O'Conell, C. & Qvon, D. (1978) Cell 15, 687-701.
26. Young, R.A. & Davis, R.W. (1983) Science 222, 778-782.
27. Young, R.A. & Davis, R.W. (1983) Proc. Natl. Acad. Sci, USA 80, 1194-1198.
28. McMaster, G.K. & Charmichael, G.G. (1977) Proc. Natl. Acad. Sci. USA 74, 48 353-48 358.
29. Thomas, P. (1980) Proc. Natl. Acad. Sci. USA 77, 5201-5205.
30. Feinberg, A.P. & Vogelstein, B. (1983) Analyt. Biochem. 132, 6-13.
31. Sanger, F., Nicklen, S. & Coulson, A.R. (1977) Proc. Natl. Acad. Sci. USA 74, 5463-5467.
32. Staden, R. (1984) Nucl, Acids Res. 12, 551-567.
33. Devereux, J., Haeberli, P. & Smithies, O. (1984) Nucl. Acids Res. 12, 387-395.
34. Wang, C.-S. & Johnson, K. (1983) Anal. Biochem. 133, 457-461.
35. Hamosh, M. (1986) in Human Milk Infant Nutrition and Health (Howell, R.R. Morriss, F.H. & Pickering, L.K. eds) pp. 66-97, Charles, C. Thomas, Springfield.
36. Wang, C.-S. (1980) Anal. Biochem. 105, 398-402.
37. Wang, C.-S & Lee, D.M. (1985) J. Lipid. Res. 26, 824-830.
38. Brenner, S. (1988) Nature 334, 528-530.
39. Yang, C.-Y., Gu, Z.-W., Yang, H.-H., Rohde, M.F., Gotto, Jr., A.M. & Pownall., H.J. (1989) J. Biol. Chem. 265, 16822-16827.
40. Han, J.H., Stratowa, C., & Rutter, W.J. (1987) Biochemistry 26, 1617-1625.
41. Kyger, E., Wiegand, R., & Lange, L. (1989) Biochim. Biophys. Res. Com 164, 1302-1309.
42. Bläckberg, L. (1981) Fat digestion in newborn infant. Umeå University Medical Dissertations New Series No 71.
43. Rudd, E.A. & Brockman, H.L. (1984) in Lipases (Borgström, B & Brockman, H.L. eds) pp. 184-204, Elsevier, Amsterdam.
44. Mickel, S., Weidenbach, F., Swarovsky, B., LaForge, S. & Scheele, G. (1989) J. Biol. Chem. 264, 12895-12901.
45. Cammulli, E.D., Linke, M.J., Brockman, H.L. & Hui, D.Y. (1989) Biochim. Biophys. Acta 1005, 177-182.
46. Mann, S.K.O. & Firtel, R.A. (1987) Mol. Cell Biol. 7, 458-469.
47. Mercken, L., Simons, M.J., Swillens, S., Masser, M. & Vassart, G. (1985) Nature 316, 647-651.
48. Lauro, R., Obici, S. Condliffe, D., Ursini, V.M., Musti, A., Moscatelli, C. & Avvedimento, V.E. (1985) Eur. J. Biochem. 148, 7-11.
49. Malthiery, Y. & Lissitzky, S. (1987) Eur, J. Biochem. 165, 491-498.
50. Prody, C., Zevin-Sonkin. D., Gnatt, A., Goldberg, O., & Soreq, H. (1987) Proc. Natl. Acad. Sci. USA 84, 3555-3559.
51. Sikorav, J-L., Krejci, E. & Massoulie', J. (1987) EMBO J. 6, 1865-1873.
52. Oakeshott, J.G. Collet, C., Phillis, R.W., Nielsen, K.M., Russel, R.J., Cambers, G.K., Ross, V. & Richmond, R.C. (1987) Proc. Natl. Acad, Sci, USA 84, 3359-3363.
53. Long, R., Satho, H., Martin, B., Kimura, S., Gonzalez, F. & Pohl, L. (1988) Biochem. Biophys. Res. Comm. 156, 866-873.
54. Hall, L.M.C. & Spierer, P. (1986) EMBO J. 5, 2949-2954.
55. Sjöberg, S., Carlsson, P., Enerbäck, S., & Bjursell, G. (1989) CABIOS 5, 41-46.
56. EP-A-317355.
57. Hernell, O., and T. Olivecrona. 1974. Human milk lipases II. Bile salt-stimulated lipase. Biochim. Biophys. Acta 369:234-244.
58. Hernell, O., L. Bläckberg, and T. Olivecrona. 1981. Human milk lipases. In Textbook of gastroenterology and nutrition in infancy. E. Lebenthal, editor. Raven Press, New York. 347-354.
59. Atkinson, S.A., M.H. Bryan, and G.H. Andersson. 1981. Human milk feeding in premature infants: protein, fat and carbohydrate balances in the first two weeks of life. J.Pediatr. 99:617-624.
60. Chappell, J.E., M.T. Clandinin, C. Kearney-Volpe, B. Reichman and P.W. Swyer. 1986. Fatty acid balance studies in premature infants fed human milk or formula: effect of calcium supplementation. J. Pediatr. 108:438-447.
61. Williamson, S., E. Finucane, H. Ellis, and H.R. Gamsu. 1978. Effect of heat treatment of human milk on absorption of nitrogen, fat, sodium, calcium and phosphorus by preterm infants. Arch. Dis. Childhood 53:555-563.

## Claims

1. The use of a recombinant protein as indicated in Figure VII from position 1 to position 722, or a functionally equivalent variant thereof, for the manufacture of a medicament for the treatment of a pathological condition related to exocrine pancreatic insufficiency.

2. The use according to claim 1 for the manufacture of a medicament for the treatment of cystic fibrosis.

3. The use according to claim 1 for the manufacture of a medicament from the treatment of chronic pancreatitis.

4. The use according to claim 1 for the manufacture of a medicament from the treatment of fat malabsorption.

5. The use according to claim 1 for the manufacture of a medicament from the treatment of malabsorption of fat soluble vitamins.

6. The use according to claim 1 for the manufacture of a medicament from the treatment of fat malabsorption due to physiological reasons.

7. The use according to any one of claims 1 to 6 wherein the said protein is in combination with a lipase or lipases or in combination with preparations containing a lipase or lipases.

## Patentansprüche

1. Verwendung eines rekombinanten Proteins, wie in Fig. VII von Position 1 bis Position 722 gezeigt, oder einer funktionell äquivalenten Variante desselben, zur Herstellung eines Medikaments zur Behandlung eines mit exokriner Pankreas-Insuffizienz verbundenen pathologischen Zustandes.

2. Verwendung nach Anspruch 1 zur Herstellung eines Medikaments zur Behandlung zystischer Fibrose.

3. Verwendung nach Anspruch 1 zur Herstellung eines Medikaments zur Behandlung chronischer Pankreatitis.

4. Verwendung nach Anspruch 1 zur Herstellung eines Medikaments zur Behandlung der Malabsorption von Fett.

5. Verwendung nach Anspruch 1 zur Herstellung eines Medikaments zur Behandlung der Malabsorption fettlöslicher Vitamine.

6. Verwendung nach Anspruch 1 zur Herstellung eines Medikaments zur Behandlung der Malabsoption von Fett, welche auf physiologische Ursachen zurückzuführen ist.

7. Verwendung nach einem der Ansprüche 1 bis 6, wobei das Protein in Verbindung mit einer Lipase oder mit Lipasen oder in Verbindung mit eine Lipase oder Lipasen enthaltenden Präparaten vorliegt.

## Revendications

1. Utilisation d'une protéine recombinante comme indiquée dans la figure VII, de la position 1 à la position 722, ou d'une variante fonctionnellement équivalente de celle-ci, pour la fabrication d'un médicament destiné au traitement d'un état pathologique lié à une insuffisance pancréatique exocrine.

2. Utilisation selon la revendication 1 pour la fabrication d'un médicament destiné au traitement de la fibrose kystique.

3. Utilisation selon la revendication 1 pour la fabrication d'un médicament destiné au traitement de la pancréatite chronique.

4. Utilisation selon la revendication 1 pour la fabrication d'un médicament destiné au traitement de la malabsorption de graisse.

5. Utilisation selon la revendication 1 pour la fabrication d'un médicament destiné au traitement de la malabsorption de vitamines liposolubles.

6. Utilisation selon la revendication 1 pour la fabrication d'un médicament destiné au traitement de la malabsorption de graisse due à des raisons physiologiques.

7. Utilisation selon l'une quelconque des revendications 1 à 6, dans laquelle ladite protéine est en combinaison avec une ou des lipases ou en combinaison avec des préparations contenant une ou des lipases.
